# EUROPÄISCHE PATENTANMELDUNG

(11) **EP 3 861 958 A1**
(43) Veröffentlichungstag der Anmeldung: **11.08.2021**
(21) Anmeldenummer: 20155980.4
(22) Anmeldetag: 06.02.2020
(51) Int. Cl.: A61B 34/30, A61B 34/00, A61B 90/50, A61B 90/00

(54) **TRAGARMGELENKVORRICHTUNG UND TRAGSYSTEM FÜR EIN MEDIZINISCHES GERÄT**

(71) Anmelder: Ondal Medical Systems GmbH, 36088 Hünfeld (DE)
(72) Erfinder: Muratidi, Georg, 36280 Oberaula (DE)
(74) Vertreter: Graf von Stosch Patentanwaltsgesellschaft mbH

(57) **Zusammenfassung**

Die Erfindung betrifft eine Tragarmgelenkvorrichtung (50) zum bewegbaren Halten eines medizinischen Geräts,
aufweisend ein erstes und zweites Befestigungselement (52, 54), welche mittels eines Federarms (56) verschwenkbar miteinander verbunden sind,
wobei der Federarm (56) einen Stützarm (58) und einen Hebelarm (60) aufweist, welche mit einer zugeordneten Stützarmschwenkachse (64) und Hebelarmschwenkachse (66) an dem ersten Befestigungselement (52) verschwenkbar gelagert sind,
wobei die beiden Schwenkachsen (64, 66) voneinander beabstandet sind,
wobei der Stützarm (58) an dem zweiten Befestigungselement (54) verschwenkbar gelagert ist und der Hebelarm (60) mittels einer Federvorrichtung (78) an dem Stützarm (58) gelagert ist, sodass eine Gewichtskraft des gehaltenen medizinischen Geräts bei unterschiedlichen Schwenkpositionen abgestützt wird,
wobei die Federvorrichtung (78) eine Verstellvorrichtung aufweist, mittels welcher eine Position der Lagerung (68, 70) des Hebelarms (60) an dem Stützarm (58) entlang einer Längserstreckung des Stützarms (58) einstellbar ist.

Weiterhin betrifft die Erfindung ein Tragarmsystem und ein Verfahren zum Betreiben einer Tragarmgelenkvorrichtung.

## Beschreibung

Die vorliegende Erfindung betrifft eine Tragarmgelenkvorrichtung für ein Tragsystem zum Halten wenigstens eines medizinischen Geräts sowie ein Tragsystem für ein medizinisches Gerät.

Tragarmsysteme werden beispielsweise in Operationssälen zum örtlichen verlagerbaren Halten eines medizintechnischen Einrichtung bzw. Geräts genutzt. Beispielsweise kann dazu ein Tragarm beweglich mittels einer Lageranordnung an einer Decke, einer Wand oder einem Boden verankert sein. Auch eine Befestigung auf einem Gestell mit Rollen ist möglich. Ein Tragsystem mit wenigstens einem Tragarm kann auch als Tragarmsystem bezeichnet werden und ein Tragsystem, welches zum Halten medizinischer Geräte ausgebildet ist, kann auch als medizinisches Tragsystem bezeichnet werden. Tragsysteme weisen üblicherweise wenigstens eine Bewegungsachse auf, damit das gehaltene medizinischen Gerät, welches auch als medizintechnisches Gerät bezeichnet werden und beispielsweise eine OP-Lampe, einen Monitor, Untersuchungsgeräte, einen oder mehrere Injektoren, OP-Besteck oder zahnmedizinische Bohrer aufweisen kann, gemäß jeweiliger Anforderungen bewegt werden kann. Die Lagerung, welche die Bewegung ermöglicht, kann auch als Gelenkvorrichtung oder Tragarmgelenkvorrichtung bezeichnet werden, wobei diese auch einen Tragarm umfassen kann. Damit kann das medizinische Gerät in eine angenehme und sichere Position zum Durchführen jeweiliger medizinischer Prozeduren und Überwachungen verbracht werden. Das örtliche Verlagern medizinischer Geräte ist bei vielen medizinischen Prozeduren essentiell, um dem medizinischen Personal die Arbeit zu erleichtern oder überhaupt zu ermöglichen.

Durch ein Schwenken eines Tragarms kann jedoch ein Hebelarm bezüglich dessen Lagerung verändert werden. Insbesondere bei einem Schwenken des Tragarms um eine im Wesentlichen horizontale Achse, wodurch die Höhe des daran gehaltenen medizinischen Geräts verändert werden kann, wird ein Hebelarm der Gewichtskraft des medizinischen Geräts gegenüber der Befestigung bzw. Lagerung des Tragarms verändert. Dennoch ist es gewünscht, dass das medizinische Gerät dabei in einer durch das Schwenken des Tragarms eingestellten Position bleibt.

Zu diesem Zweck werden häufig federbalancierte Tragarme oder Tragarmgelenkvorrichtungen genutzt. Dabei werden zwei voneinander beabstandete Arme vorgesehen, welche sich mittels einer Feder gegenseitig stützen. Durch Verschwenken des federbalancierten Tragarms ändert sich dabei der Hebelarm der Federkraft korrespondierend zu dem Hebelarm der Traglast, wodurch das medizinische Gerät in unterschiedlichen Positionen gleichermaßen gut gehalten werden kann. Alternativ oder zusätzlich kann auch die Feder durch das Schwenken komprimiert oder gestreckt werden, wodurch ebenfalls eine korrespondierende Stützkraft geändert werden kann.

Eine solche Tragarmgelenkvorrichtung ist beispielsweise in der EP 3 217 939 A1 gezeigt. Dort kann der Abstand zwischen einer Lagerung von zwei Armen eingestellt werden, um unterschiedliche große Lasten gleichermaßen abstützen bzw. in Position halten zu können. Das Verschwenken der dort gezeigten Tragarmgelenkvorrichtung selber erfolgt direkt manuell, was eine Erreichbarkeit für den Benutzer voraussetzt.

Bekannt ist es auch, Bremsen zu verwenden, welche einen Tragarm in Position halten. Bremsen haben jedoch den Nachteil, dass sie verschleißen. Zudem ist auch eine Lastanpassung bei Bremsen gegebenenfalls notwendig.

Weiterhin bekannt ist ein direktes Verschwenken mittels eines Motors, welcher an einer Lagerung eines Tragarms angeordnet ist. Dafür kann jedoch ein großer und starker Motor notwendig sein. Ein Beispiel für solch eine Technologie wird später noch anhand von Figuren detaillierter erläutert.

Aufgabe der vorliegenden Erfindung ist es, eine verbesserte Tragarmgelenkvorrichtung, ein verbessertes Tragarmsystem und ein verbessertes Verfahren zum Betreiben einer Tragarmgelenkvorrichtung bereitzustellen. Insbesondere soll ein kostengünstiges Halten von medizinischen Geräten in einer präzise einstellbaren Stellung einfach ermöglicht werden.

Diese Aufgaben werden erfindungsgemäß durch die Gegenstände der unabhängigen Patentansprüche gelöst. Vorteilhafte Ausgestaltungen mit zweckmäßigen Weiterbildungen der Erfindung sind in den jeweiligen Unteransprüchen angegeben, wobei vorteilhafte Ausgestaltungen eines Aspekts als vorteilhafte Ausgestaltungen jeweiliger anderer Aspekte und umgekehrt anzusehen sind.

Ein erster Aspekt der Erfindung betrifft eine Tragarmgelenkvorrichtung für ein Tragsystem zum bewegbaren Halten wenigstens eines medizinischen Geräts. Nach einem Bewegen des Geräts kann dabei dessen Position bzw. Stellung gehalten werden, was auch als Fixierung in der Stellung bzw. Position bezeichnet werden kann.

Die Tragarmgelenkvorrichtung weist ein erstes und ein zweites Befestigungselement auf, welche mittels eines Federarms relativ verschwenkbar zueinander miteinander verbunden sind. Der Federarm ist dabei auch Teil der Tragarmgelenkvorrichtung und ermöglicht durch ein Verschwenken wenigstens um eine Schwenkachse, bevorzugt in einer Schwenkebene, die beiden Befestigungselemente relativ zueinander zu bewegen. Eines der beiden Befestigungselemente, bevorzugt das erste Befestigungselement, kann ortsfest sein, beispielsweise durch eine Verankerung an einer Wand. Das andere Befestigungselement kann so vorzugsweise relativ zu dieser Verankerung verschwenkt werden, insbesondere um dessen Höhe zu ändern.

Der Federarm kann einen Stützarm und einen Hebelarm aufweisen, welche mit einer zugeordneten Stützarmschwenkachse und Hebelarmschwenkachse an dem ersten Befestigungselement verschwenkbar gelagert sind. Diese beiden Schwenkachsen sollten voneinander beabstandet sein, wobei die Schwenkachsen bevorzugt parallel zueinander angeordnet sind. Der Stützarm und der Hebelarm werden vorzugsweise bei einer Schwenkbewegung des Federarms bzw. beim Verschwenken der beiden Befestigungselemente relativ zueinander ebenfalls verschwenkt. Wenigstens eines der beiden Befestigungselemente ist gelenkig an dem Federarm angeordnet, insbesondere das erste Befestigungselement gelenkig sowohl an dem Stützarm als auch an dem Hebelarm. Bevorzugt ist das andere Befestigungselement, insbesondere das zweite Befestigungselement, ebenfalls gelenkig an dem Federarm gelagert, vorzugsweise ausschließlich an dem Stützarm.

Der Stützarm ist bevorzugt an dem zweiten Befestigungselement verschwenkbar gelagert. Der Hebelarm ist dagegen beispielsweise mittels einer Federvorrichtung an dem Stützarm derart gelagert ist, dass eine Gewichtskraft des gehaltenen medizinischen Geräts bei unterschiedlichen Schwenkpositionen abgestützt wird. Durch die Federvorrichtung und die beabstandete Lagerung von Stützarm und Hebelarm kann sich eine Stützkraft der Tragarmgelenkvorrichtung automatisch an deren Schwenkwinkel bzw. deren Schwenkposition anpassen, um die Gewichtskraft gleichermaßen bei unterschiedlichen Hebelarmlängen der Gewichtskraft abzustützen. Die Tragarmgelenkvorrichtung ist so selbstbalancierend und/oder unterstützt ein Verstellen des Schwenkwinkels gegen die Schwerkraft.

Zusätzlich zu der Gewichtskraft des medizinischen Geräts kann so auch das Eigengewicht jeweiliger Komponenten der Tragarmgelenkvorrichtung abgestützt bzw. ausbalanciert werden. Beispielsweise wirkt auch eine Gewichtskraft eines nicht verankerten der beiden Befestigungselemente mit unterschiedlichen Hebelarmen, womit unterschiedliche Drehmomente je nach Stellung der Tragarmgelenkvorrichtung abgestützt werden sollte.

Im Rahmen dieser Anmeldung betrifft der Hebelarm ein Bauteil der Tragarmgelenkvorrichtung, welcher beispielsweise als Stab, Stange Rohr, Hohlprofil oder dergleichen ausgebildet sein kann. Dieser Hebelarm stützt das zu haltende medizinische Gerät mit ab. Hebelarme im Kontext von Kräften bezeichnen dagegen den Abstand eines Kraftangriffspunkts von einem Drehgelenk bzw. Drehpunkt. Solche Hebelarme bezeichnen also eine Länge, durch deren Multiplikation mit der jeweiligen Kraft sich das an dem Drehpunkt wirkende Drehmoment, häufig auch als Moment bezeichnet, berechnen lässt.

Die Federvorrichtung kann eine Verstellvorrichtung aufweisen, mittels welcher eine Position der Lagerung des Hebelarms an dem Stützarm entlang einer Längserstreckung des Stützarms einstellbar ist. Durch diese Positionsverstellung wird also eine Position verstellt, an welcher der Hebelarm an dem Stützarm angreift. Alternativ oder zusätzlich kann so auch ein Schwenkwinkel des Hebelarms bezüglich der Hebelarmschwenkachse eingestellt werden. Insgesamt ergibt sich durch diese Verstellung ein Schwenkwinkel der Tragarmgelenkvorrichtung bzw. eine relative Position der beiden Befestigungselemente zueinander. Durch Verschieben der Lagerung des Hebelarms am Stützarm und/oder ein Verschieben eines Kraftangriffspunkts kann also ein Schwenkwinkel der Tragarmgelenkvorrichtung bzw. des Federarms eingestellt werden.

Vorzugsweise ist die Verstellvorrichtung dazu ausgebildet, dass sich ein Spannungszustand des Federarms bzw. der Federvorrichtung durch ein Verstellen der Lagerung des Hebelarms an dem Stützarm ändert. Beispielsweise kann entsprechend eine Feder komprimiert oder entspannt werden. Bevorzugt ist die Federvorrichtung dabei dazu ausgebildet, eine frei werdende potentielle Energie bei einem Absenken der Tragarmgelenkvorrichtung bzw. des daran gehaltenen medizinischen Geräts zu speichern und beim Anheben der Tragarmgelenkvorrichtung bzw. des daran gehaltenen medizinischen Geräts wieder abzugeben. Beispielsweise kann beim Absenken des Federarms durch Verstellen der Lagerung des Hebelarms an dem Stützarm eine Feder der Federvorrichtung komprimiert werden und beim Anheben des Federarms durch Verstellen der Lagerung des Hebelarms an dem Stützarm eine Feder der Federvorrichtung entspannt werden. Entsprechend stützt die Feder beim Absenken, wodurch geringere Stützkräfte aufgebracht werden müssen. Beim Anheben gibt die Feder die beim Absenken gespeicherte Energie wieder ab, wodurch ein Kraftaufwand zum Anheben geringer ist. Dadurch ist eine präzise Positionseinstellung mit geringem Kraftaufwand und einer simplen Mechanik einfach möglich. Gegebenenfalls muss sogar bei der Abwärtsbewegung eine Kraft zusätzlich zum Eigengewicht aufgebracht werden, um den Federarm entgegen der stützenden Federkraft zu bewegen. Die Tragarmgelenkvorrichtung, insbesondere die Federvorrichtung, kann so ausgebildet sein, dass beim Absenken und Anheben annähernd die gleichen Kräfte benötigt werden. Damit ist die Bedienung und Positionseinstellung besonders intuitiv und präzise möglich.

Die Tragarmgelenkvorrichtung erlaubt also einen balancierten Tragarm mit einem einstellbaren Schwenkwinkel bereitzustellen. Durch das Einstellen an der Federvorrichtung kann dabei auch ein Ziehen an irgendeiner Komponente zum Verstellen verzichtet werden und stattdessen eine mechanische Einstellbarkeit bereitgestellt werden. Diese erlaubt ein wesentlich präziseres und/oder schnelleres Einstellen, insbesondere durch eine Untersetzung oder Übersetzung beispielsweise an einem Einstellelement, wie einem Drehknopf.

Auch kann die Verstellvorrichtung selbsthemmend, selbstblockierend und/oder arretierend ausgebildet sein. Beispielsweise kann eine Verstellschraube, wie eine Gewindestange, vorgesehen werden, welche durch eine Gewindesteigung ein Verstellen der Position der Lagerung des Hebelarms durch Gewichtskräfte des medizinischen Geräts verhindert. Dadurch wird dessen Position präzise gehalten. Auch ein Nachschwingen nach einer Verstellung kann so wirksam verhindert oder zumindest reduziert werden.

Insbesondere kann so auch ein motorisch und/oder mechanisch verstellbarer Federarm im Bereich von leichten und mittelgroßen Lasten zur Verfügung gestellt werden. Der Aufbau ist einfach und kostengünstig. Die Verstellvorrichtung kann für eine stufenlose Verstellung ausgebildet sein, insbesondere eine stufenlose Positionierung und/oder Höhenverstellung. Auch bei Traglastverlusten, beispielsweise durch ein Abmontieren des medizinischen Geräts, und/oder zusätzliche Lasten, beispielsweise ein Drücken auf Bedienfelder des medizinischen Geräts, kann der Federarm in Position bleiben, insbesondere durch die beschriebene Selbsthemmung.

Die Federvorrichtung kann beispielsweise eine vorgespannte Feder aufweisen, welche beispielsweise bereits einen großen Teil der wirkenden Lasten trägt bzw. ausbalanciert. Ein Verstellen benötigt so nur noch sehr geringe Kräfte, womit beispielsweise eine geringe Bedienkraft und/oder nur ein kleiner Motor notwendig ist/sind. Vorzugsweise ist die Feder als Schraubenfeder und/oder Druckfeder ausgebildet. Auch die Gefahr durch mögliche Kollisionen bei einer Fehlbedienung ist so geringer, da die dann wirkenden Bedienkräfte ebenfalls gering ist. Bei einem Einsatz als Teil eines Robotikarms können so beispielsweise deutlich weniger Risikovermeidungsmaßnahmen notwendig sein.

Die jeweiligen Befestigungselemente können beispielsweise zum Halten des medizinischen Geräts, eines Tragarms und/oder einer weiteren Tragarmgelenkvorrichtung ausgebildet sein. Bevorzugt ist das medizinische Gerät an dem zweiten Befestigungselement befestigt. Das medizinische Gerät kann beispielsweise ein Monitor, ein EKG-Gerät, ein Gerät für Infusionen und/oder Operationen sein, Teil einer Bohrvorrichtung für Zahnärzte oder auch eines Tabletts zum Halten medizinischer Werkzeuge, wie Skalpelle und dergleichen, sein. Eines der Befestigungselemente kann beispielsweise zur Befestigung an einer Wand, einer Decke, einem Boden und/oder einer beweglichen oder stationären Basiseinheit ausgebildet sein. Die Befestigungselemente können beispielsweise eine Kopplung, wie jeweilige Schrauben und/oder Schnellspanner, zum Befestigen von jeweiligen Teilen des Tragarmsystems daran, wie beispielsweise weiteren Tragarmen und/oder des medizinischen Geräts, aufweisen. Eines oder beide der Befestigungselemente kann/können auch zum Koppeln mit einem Tragarm ausgebildet sein, insbesondere zu einem bewegbaren Koppeln. Beispielsweise kann das jeweilige Befestigungselement hierzu ein Lager und/oder ein Gelenk aufweisen. Dadurch kann das Befestigungselemente und damit auch das medizinische Gerät relativ zu dem Federarm und/oder dem anderen Befestigungselement ausgerichtet werden, beispielsweise durch Drehung in einer vertikalen oder horizontalen Ebene. Insgesamt ergibt sich eine flexible und/oder modulare Gestaltung.

Der Hebelarm und der Stützarm können als längliche Bolzen, Stange, Stab, Zylinder, Hohlprofile, Massivprofile und/oder ähnliches ausgebildet sein. Als Materialien kommen beispielsweise Kunststoff oder Metall zum Einsatz. Der Hebelarm und der Stützarm können einteilig oder mehrteilig ausgebildet sein. Vorzugsweise sind der Hebelarm und der Stützarm mit einem jeweiligen Ende an deren zugeordneten Stützarmschwenkachse bzw. Hebelarmschwenkachse gelagert, also mit dem jeweiligen Ende an dem ersten Befestigungselement. Mit seinem gegenüberliegenden Ende ist der Stützarm bevorzugt an dem zweiten Befestigungselement gelagert, insbesondere schwenkbar oder drehfest. Der Hebelarm ist mit seinem gegenüberliegenden Ende bevorzugt an der der Federvorrichtung, insbesondere der Verstellvorrichtung, und/oder an dem Stützarm gelagert. Diese Lagerung sollte axial verstellbar sein, um die Schwenkverstellung. Vorzugsweise ist die Lagerung dabei axial entlang der Längserstreckung des Stützarms durch die Federvorrichtung abgestützt, um jeweilige zum Verstellen notwendige Kräfte mittels der Federvorrichtung stützen zu können.

Der Hebelarm ist mittels der Federvorrichtung vorzugsweise von dem zweiten Befestigungselement beabstandet an dem Stützarm gehalten. Der Hebelarm kann dabei mittelbar über die Federvorrichtung bzw. Verstellvorichtung an dem Stützarm gehalten sein oder auch direkt an diesem gelagert sein, wobei die Verstellvorrichtung eine Bewegung der Lagerung bewirkt und/oder die Federvorrichtung einen Kraftfluss von dem Hebelarm zu dem Stützarm ermöglicht, insbesondere entlang der axialen Erstreckung des Stützarms.

Das eine der beiden Befestigungselemente kann mittels des Federarms relativ zu dem anderen der beiden Befestigungselemente örtlich verlagert werden, insbesondere in einer Hoch- bzw. Vertikalrichtung. Alternativ oder zusätzlich kann mittels Tragarmgelenkvorrichtung, insbesondere mittels des Federarms, auch eine örtliche Verlagerung in einer Quer- bzw. Horizontalrichtung ermöglicht werden. Vorzugsweise ist dabei nur eine Schwenkebene, insbesondere eine vertikale ausgerichtete Schwenkebene für das Verstellen in Hochrichtung, mittels des Federarms ausbalanciert. Beispielsweise sind die jeweiligen Befestigungselemente an gegenüberliegenden Enden des Federarms an diesem angeordnet, insbesondere befestigt.

In weiterer vorteilhafter Ausgestaltung der Tragarmgelenkvorrichtung ist es vorgesehen, dass die Federvorrichtung eine Feder aufweist. Eine Feder ist ein einfaches Element zum Aufnehmen bzw. Speichern und Abgeben potentieller Energie, womit ein Ausbalancieren mit einfachen Mitteln möglich ist und/oder eine Verstell- bzw. Bedienkraft besonders klein sein kann. Beispielsweise ist eine hydraulische oder mechanische Feder geeignet, insbesondere eine Gasdruckfeder und/oder eine Schraubenfeder. Eine mechanische Feder ist besonders kostengünstig und robust. Zusätzlich kann die Federvorrichtung auch eine Dämpfung aufweisen, welche beispielsweise ein plötzliches Hochschnellen bei Entlastung, z.B. durch Abnahme des medizinischen Geräts, verhindert. Diese Dämpfung kann besonders einfach in einer hydraulischen Feder integriert sein. Vorzugsweise ist die Feder im Wesentlichen innerhalb des Stützarms geführt und/oder angeordnet. Dadurch ist die Feder vor Umwelteinflüssen geschützt angeordnet und/oder ein Verletzungsrisiko durch Fehlbedienung, beispielsweise ein Greifen der Feder, kann minimiert werden. Im Wesentlichen bedeutet hier insbesondere, dass die Feder je nach Position der Tragarmgelenkvorrichtung bzw. des Federarms und/oder der Lagerung des Hebelarms, teilweise aus dem Stützarm ragen kann. Bevorzugt ist die Feder in jeder Position vollständig im Stützarm aufgenommen. Der Stützarm kann hierzu ein Federrohr umfassen oder als solches ausgebildet sein.

In weiterer vorteilhafter Ausgestaltung der Tragarmgelenkvorrichtung ist es vorgesehen, dass der Hebelarm verschwenkbar an der Federvorrichtung gelagert ist, insbesondere mit einer Schwenkachse parallel zu der Hebelarmschwenkachse, mittels welcher der Hebelarm an dem ersten Befestigungselement gelagert ist. Die Position dieser Schwenkachse kann axial entlang des Stützarms verschiebbar sein. Eine entsprechende Lagerung kann auch als Hebellager bezeichnet werden. Bevorzugt ist der Hebelarm innerhalb des Stützarms und/oder einer Verkleidung gelagert, um ein Quetschungsrisiko zu reduzieren und Beschädigungen zuverlässig zu vermeiden.

In weiterer vorteilhafter Ausgestaltung der Tragarmgelenkvorrichtung ist es vorgesehen, dass der Hebelarm mittels eines Schlittens an dem Stützarm entlang dessen Längserstreckung gelagert ist, wobei der Schlitten vorzugsweise im Wesentlichen innerhalb des Stützarms geführt ist. Insbesondere kann der Schlitten axial beweglich an oder in dem Stützarm an diesem gelagert sein. In der einfachsten Form ist der Schlitten an einen Innendurchmesser eines Rohrs des Stützarms angepasst und so geführt. Insbesondere kann der Hebelarm verschwenkbar an dem Schlitten gelagert sein. Der Schlitten erlaubt eine einfache Führung des Hebelarms bei einer axialen Verstellbarkeit und Kraftübertragung an die bzw. von der Federvorrichtung. Die Anordnung innerhalb erlaubt auch hier den Schutz der Teile und von Personen. Insbesondere eine Verschmutzung und damit frühzeitiger Verschleiß kann so vermieden werden. Der Schlitten kann Teil der Verstellvorrichtung sein und wird axial am Stützarm beim Einstellen der Position der Lagerung verschoben. Der Schlitten kann, je nach Position, teilweise aus dem Stützarm ragen oder in jeder Position vollständig darin aufgenommen sein.

In weiterer vorteilhafter Ausgestaltung der Tragarmgelenkvorrichtung ist es vorgesehen, dass die Verstellvorrichtung eine Gewindestange aufweist, durch deren Verschieben die Position der Lagerung des Hebelarms am Stützarm einstellbar ist. Das Verschieben der Gewindestange kann dabei relativ zu dem Stützarm erfolgen. Das Verschieben kann beispielsweise durch eine Drehung der Gewindestange verursacht werden. Durch die Drehung der Gewindestange kann demnach die Position der Lagerung des Hebelarms am Stützarm eingestellt werden. Die Gewindestange erlaubt ein konstruktiv einfach vorgebbares Übersetzungsverhältnis, insbesondere eine Untersetzung. So kann die Verstellung der Tragarmgelenkvorrichtung mit geringer Kraft bzw. geringem Drehmoment erfolgen. Zudem kann eine Gewindestange einfach selbsthemmend sein, sodass bei Ausfall eines Antriebs, insbesondere des Motors, und/oder einem defekt der Feder ein Tragarm bzw. das daran gehaltene medizinische Gerät nicht einfach herunterfällt. Außerdem kann die Gewindestange auch hohe Kräfte zuverlässig aufnehmen. Vorzugsweise ist die Gewindestange im Wesentlichen innerhalb des Stützarms angeordnet, wobei, je nach Stellung, die Gewindestange teilweise aus dem Stützarm ragen oder in jeder Position bzw. Stellung vollständig darin aufgenommen sein kann.

In weiterer vorteilhafter Ausgestaltung der Tragarmgelenkvorrichtung ist es vorgesehen, dass die Gewindestange an einer axial befestigten und drehbaren Mutter gelagert ist, welche in oder an dem Stützarm befestigt ist. Eine Drehung der Mutter bewirkt dann eine axiale Verstellung der Position der Gewindestange. Die Mutter kann insbesondere als Spindelmutter ausgebildet sein. Bevorzugt ist die Gewindestange an dem Schlitten und/oder dem Hebelarm befestigt, insbesondere mit einem Ende, bevorzugt einem der Mutter abgewandten Ende. Die Mutter kann in ihrer axialen Position entlang der Längserstreckung des Stützarms festgelegt befestigt sein, beispielsweise durch ein Anschlagelement, welches in einer korrespondierenden Nut der Mutter und/oder einer Nut oder Aufnahme des Stützarms angeordnet ist. Insgesamt kann mit der Gewindestange und optional mit der Mutter eine einfache, robuste, zuverlässige, kostengünstige und wartungsarme Verstellvorrichtung bereitgestellt werden.

In weiterer vorteilhafter Ausgestaltung der Tragarmgelenkvorrichtung ist es vorgesehen, dass die Verstellvorrichtung ein Stellelement aufweist, mittels welchem die Position der Lagerung des Hebelarms an dem Stützarm einstellbar ist, wobei das Stellelement bevorzugt an dem Stützarm gelagert ist. Das Stellelement kann beispielsweise eine manuelle Verschiebung der Lagerung des Hebelarms an dem Stützarm bewirken, womit entsprechend manuell ein Schwenkwinkel präzise einstellbar ist. Das Stellelement kann also für eine manuelle Betätigung und/oder als Drehknopf ausgebildet sein. Bevorzugt ist das Stellelement mittels eines Getriebes, wie beispielsweise korrespondierender Kegelräder, mit der Gewindestange und/oder der Mutter im Eingriff. Bevorzugt ist eine Betätigungs- bzw. Drehachse des Stelleelements quer, insbesondere orthogonal, zur Längserstreckung des Stützarms ausgerichtet. Jeweilige Kegelräder erlauben es, ein Übersetzungsverhältnis einfach vorzugeben und/oder die Betätigungsachse der Verstellvorrichtung und des Stellelements quer, insbesondere orthogonal, zueinander auszurichten. Dadurch kann die Tragarmgelenkvorrichtung besonders kompakt ausgebildet sein.

In weiterer vorteilhafter Ausgestaltung der Tragarmgelenkvorrichtung ist es vorgesehen, dass die Verstellvorrichtung einen Motor aufweist, mittels welchem die Position der Lagerung des Hebelarms an dem Stützarm einstellbar ist, wobei der Motor bevorzugt an dem Stützarm gelagert ist. Insbesondere kann der Motor in dem Stützarm wenigstens teilweise oder komplett und/oder in einer axialen Verlängerung des Stützarms angeordnet sein, wodurch die Vorrichtung kompakt und robust sein kann. Mit dem Motor wird also ein Antrieb der Verstellung der Tragarmgelenkvorrichtung bereitgestellt. Dadurch ist eine Automation und/oder Regelung möglich, insbesondere kann die Tragarmgelenkvorrichtung so einfach als Teil eines robotischen Tragarmsystems genutzt werden. Der Motor ist bevorzugt als Elektromotor ausgebildet. Beispielsweise kann der Motor zum Drehen der Mutter und/oder der Gewindestange ausgebildet sein. Der Motor erlaubt eine elektrische Steuerung des Schwenkwinkels der Tragarmgelenkvorrichtung bzw. der beiden Befestigungselemente relativ zueinander. Bevorzugt ist der Motor als Getriebemotor ausgebildet.

In weiterer vorteilhafter Ausgestaltung der Tragarmgelenkvorrichtung ist es vorgesehen, dass die Tragarmgelenkvorrichtung eine Sensorvorrichtung aufweist, welche dazu ausgebildet ist, eine auf die Tragarmgelenkvorrichtung wirkende Kraft und/oder eine Stellung der beiden Befestigungselemente relativ zueinander zu erfassen. Diese Erfassung kann beispielsweise auch einfach dadurch erfolgen, dass ein Schwenkwinkel des Stützarms und/oder des Hebelarms bezüglich der Stützarmschwenkachse und/oder der Hebelarmschwenkachse erfasst wird, beispielsweise durch einen Winkelmesser. Alternativ oder zusätzlich ist beispielsweise ein Beschleunigungsmesser oder eine bildliche Erfassung geeignet.

Zudem kann die Tragarmgelenkvorrichtung eine Steuervorrichtung aufweist, welche in Abhängigkeit von der erfassten auf die Tragarmgelenkvorrichtung wirkende Kraft und/oder der erfassten Stellung der Tragarmgelenkvorrichtung den Motor zum Halten der relativen Stellung der beiden Befestigungselemente zueinander steuert. Die erfasste Stellung der Tragarmgelenkvorrichtung kann dabei die relative Position bzw. Stellung der beiden Befestigungselemente zueinander mit umfassen oder als diese definiert sein. So kann eine automatische Steuerung zum Einnehmen und/oder Halten bestimmter Stellungen implementiert werden. Insbesondere ist so eine Regelung möglich, um die Position des medizinischen Geräts zu halten. Die Tragarmgelenkvorrichtung kann so äußere Kräfte, beispielsweise durch ein Drücken eines Betätigungselements des medizinischen Geräts, kompensieren. Dies ermöglicht ein präzises Halten der Position, sodass ein entsprechendes Tragarmsystem auch bei nur teilrobotisch durchgeführten OPs gut genutzt werden kann, ohne dass durch ein versehentliches Berühren bzw. Belasten jeweiliger Tragarme eine Gefahr eines unbeabsichtigten Verstellens droht.

In weiterer vorteilhafter Ausgestaltung der Tragarmgelenkvorrichtung ist es vorgesehen, dass die Tragarmgelenkvorrichtung ein Parallelführungselement aufweist, welches jeweils schwenkbar an dem ersten Befestigungselement und dem zweiten Befestigungselement beabstandet zu dem Stützarm gelagert ist, insbesondere dessen Lagerung an dem jeweiligen Befestigungselement. Vorzugsweise sind dabei jeweilige Lagerachsen des Parallelführungselements parallel zueinander und/oder zu jeweiligen Lagerachsen des Stützarms. Mittels des Parallelführungselements kann eine Ausrichtung des gehaltenen medizinischen Geräts bei Verschwenken der Tragarmgelenkvorrichtung bzw. des Federarms beibehalten werden. Das Parallelführungselement ist beispielsweise dazu ausgebildet, eine Ausrichtung des zweiten Befestigungselements an eine Schwenkbewegung der beiden Befestigungselemente zueinander zu koppeln. So kann beispielsweise ein Monitor in einer gewählten Ausrichtung, beispielsweise einer horizontalen Ausrichtung, auch bei einer Höhenverstellung dessen Halterung beibehalten werden. Bevorzugt ist das Parallelführungselement als Parallelführungsstange ausgebildet. Mittels des Parallelführungselements kann eine Kopplung des Schwenkwinkels des ersten Befestigungselements mit dem Schwenkwinkel des zweiten Befestigungselements bereitgestellt werden.

In weiterer vorteilhafter Ausgestaltung der Tragarmgelenkvorrichtung ist es vorgesehen, dass die Tragarmgelenkvorrichtung dazu ausgebildet ist, eine Stützkraft an unterschiedliche Gewichtskräfte unterschiedlicher gehaltener medizinischer Geräte bei unterschiedlichen Schwenkpositionen anzupassen, insbesondere durch Austausch der Feder der Federvorrichtung, Einstellen einer Vorspannkraft der Federvorrichtung und/oder Einstellen eines Abstands zwischen der Hebelarmschwenkachse und der Stützarmschwenkachse an dem ersten Befestigungselement. Durch den Abstand zwischen der Hebelarmschwenkachse und der Stützarmschwenkachse kann ein Hebelarm der Stützkraft und damit ein stützendes Drehmoment eingestellt werden. Ebenso kann die Stützkraft durch eine Vorspannung einer Feder angepasst werden. Die Einstellbarkeit der Stützkraft ermöglicht eine besonders variabel einsetzbare Tragarmgelenkvorrichtung. Der Austausch bzw. das Vorsehen einer an jeweiligen Nutzlasten angepasster Federn ermöglicht eine besonders kostengünstige und robuste Bauweise.

In weiterer vorteilhafter Ausgestaltung der Tragarmgelenkvorrichtung ist es vorgesehen, dass das erste Befestigungselement und/oder das zweite Befestigungselement zur Befestigung an einer Wand, einer Decke, einem Boden, einer beweglichen und/oder einer stationären Basiseinheit ausgebildet ist und/oder das erste Befestigungselement und/oder das zweite Befestigungselement zur Kopplung mit einem Tragarm, einem Gelenk, einer weiteren Tragarmgelenkvorrichtung und/oder dem bzw. einem medizinischen Gerät ausgebildet ist. So kann eine modulare Bauweise ermöglicht werden. Zudem kann so ein Schwerpunkt der Tragarmgelenkvorrichtung in Richtung einer festen Verankerung verlagert werden, wodurch das Eigengewicht der Tragarmgelenkvorrichtung mit geringeren Kräften gestützt bzw. kompensiert werden kann.

Ein zweiter Aspekt der Erfindung betrifft ein Tragarmsystem zum bewegbaren Halten wenigstens eines medizinischen Geräts. Dieses Tragsystem weist eine Tragarmgelenkvorrichtung gemäß dem ersten Aspekt der Erfindung auf. Die Tragarmgelenkvorrichtung kann dabei auch als Tragarm betrachtet werden, insbesondere deren Federarm. Das Tragsystem kann ein oder mehrere Tragarme und/oder Tragarmgelenkvorrichtung aufweisen, welche miteinander gekoppelt sein können, insbesondere mittels jeweiliger Befestigungselemente. Dabei kann pro zwei Tragarme und/oder Tragarmgelenkvorrichtung ein gemeinsames Befestigungselement vorgesehen sein, mittels welchem diese miteinander verbunden bzw. gekoppelt sind. Die sich aus der Tragarmgelenkvorrichtung gemäß dem ersten Erfindungsaspekt ergebenden Merkmale und Vorteile sind den Beschreibungen des ersten Erfindungsaspekts zu entnehmen, wobei vorteilhafte Ausgestaltungen des ersten Erfindungsaspekts als vorteilhafte Ausgestaltungen des zweiten Erfindungsaspekts und umgekehrt anzusehen sind.

Das Tragarmsystem kann beispielsweise als Deckenversorgungseinheit, Wandhalterung, Monitorträger und/oder als Teil einer Mobillösung ausgebildet sein. Das Tragarmsystem kann auch eine Basiseinheit, eine stationäre Befestigung, ein oder mehrere miteinander verbundene Tragarme und/oder wenigstens ein mittels des Tragarmsystems gehaltenes medizinisches Gerät aufweisen.
Bevorzugt ist das medizinische Gerät mittels wenigstens der Tragarmgelenkvorrichtung bewegbar, insbesondere in Hochrichtung bzw. in einer vertikalen Richtung durch eine Schwenkbewegung des Federarms verstellbar gehalten, insbesondere durch Schwenken des Stützarms bezüglich des ersten und/oder des zweiten Befestigungselements.

Ein dritter Aspekt der Erfindung betrifft ein Verfahren zum Betreiben der Tragarmgelenkvorrichtung und/oder des Tragarmsystems gemäß dem ersten bzw. zweiten Aspekts, wobei die Verstellvorrichtung wenigstens den Motor aufweist, mittels welchem die Position der Lagerung des Hebelarms an dem Stützarm einstellbar ist. Zusätzlich können auch die Sensorvorrichtung und die Steuervorrichtung vorgesehen sein. Die sich aus der Tragarmgelenkvorrichtung gemäß dem ersten Erfindungsaspekt und dem Tragarmsystem gemäß dem zweiten Erfindungsaspekt ergebenden Merkmale und Vorteile sind den Beschreibungen des ersten bzw. zweiten Erfindungsaspekts zu entnehmen, wobei vorteilhafte Ausgestaltungen des ersten und des zweiten Erfindungsaspekts als vorteilhafte Ausgestaltungen des dritten Erfindungsaspekts und umgekehrt anzusehen sind.

Für das Betreiben wird die Tragarmgelenkvorrichtung und/oder des Tragarmsystems bereitgestellt, insbesondere mit daran befestigtem medizinischem Gerät. Es erfolgt zudem ein Erfassen einer auf die Tragarmgelenkvorrichtung wirkende Kraft und/oder einer Stellung der beiden Befestigungselemente relativ zueinander. In Abhängigkeit von der erfassten auf die Tragarmgelenkvorrichtung wirkende Kraft und/oder der erfassten Stellung der Tragarmgelenkvorrichtung wird zum Halten der relativen Stellung der beiden Befestigungselemente zueinander der Motor der Tragarmgelenkvorrichtung gesteuert.

Beispielsweise kann sich so eine Anwendung im Bereich der Injektoren verbessert werden. Durch das Entleeren von Flüssigkeit aus dem Injektor wird eine angehängte Traglast reduziert. Normalerweise würde sich dadurch ein Federarm nicht mehr im Gleichgewicht befinden und nach oben bewegen. Durch den einstellbaren Federarm kann dieser jedoch in Position gehalten werden, sodass die Anwendung am Patienten erfolgen kann.

Auch eine Anwendung im Bereich eines Dokumentationsterminals ist so gut möglich, bei welchem beispielsweise ein Monitor und eine Tastatur gehalten ist. Mit der Bedienung der Tastatur wird eine zusätzliche Bedienkraft durch das Tippen eingebracht, wodurch sich der Federarm nach unten bewegen könnte. Auch dies kann durch den Antrieb kompensiert werden.

Ansonsten ermöglicht der Antrieb auch eine exakte Positionierung, beispielsweise eines Mikroskops gesteuert beispielsweise in Abhängigkeit von einem dreidimensionalen Scan eines Patienten.

Jeweilige Gewichtskraftänderungen können alternativ oder zusätzlich auch durch die Mechanik der Verstellung gehemmt aufgenommen werden, welche beispielsweise durch ihre Reibung bei geringfügigen Belastungsänderungen des Federarms dessen Verstellen verhindern.

Weitere Merkmale der Erfindung ergeben sich in den Ansprüchen, den Ausführungsbeispielen sowie anhand der Zeichnungen. Die vorstehend in der Beschreibung genannten Merkmale und Merkmalskombinationen sowie die nachfolgend in den Ausführungsbeispielen genannten Merkmale und Merkmalskombinationen sind nicht nur in der jeweils angegebenen Kombination, sondern auch in anderen Kombinationen verwendbar, ohne den Rahmen der Erfindung zu verlassen.

Dabei zeigt:
- Fig. 1: in einer schematischen Seitenansicht eine Tragarmgelenkvorrichtung gemäß dem Stand der Technik in einer waagerechten Position;
- Fig. 2: in einer schematischen Seitenansicht die Tragarmgelenkvorrichtung gemäß Fig. 1 in einer unteren Position;
- Fig. 3: in einer schematischen seitlichen Schnittansicht eine erfindungsgemäße Tragarmgelenkvorrichtung in einer waagerechten Position;
- Fig. 4: in einer schematischen seitlichen Schnittansicht die Tragarmgelenkvorrichtung gemäß Fig. 3 in einer unteren Position;
- Fig. 5: in einer schematischen seitlichen Schnittansicht die Tragarmgelenkvorrichtung gemäß Fig. 3 in einer oberen Position;
- Fig. 6: in einer schematischen Perspektivansicht ein Detail einer Ausführungsform der Tragarmgelenkvorrichtung gemäß Fig. 3;
- Fig. 7: das in Fig. 6 gezeigte Detail in einer schematischen geschnittenen Perspektivansicht.
- Fig. 8: in einer schematischen Perspektivansicht ein Detail einer weiteren Ausführungsform der Tragarmgelenkvorrichtung gemäß Fig. 3;
- Fig. 9: das in Fig. 8 gezeigte Detail in einer schematischen seitlichen Schnittansicht;

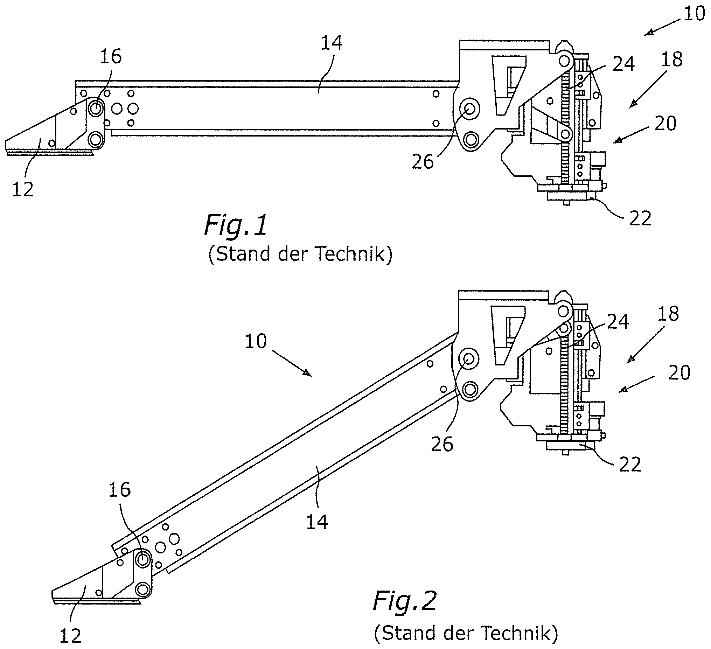

Fig. 1 zeigt in einer schematischen Seitenansicht eine Tragarmgelenkvorrichtung 10 gemäß dem Stand der Technik. Diese weist ein erstes Befestigungselement 12 auf, mittels welchem die Tragarmgelenkvorrichtung 10 beispielsweise eine OP-Lampe halten kann. An diesem Befestigungselement 12 ist ein Tragarm 14 angeordnet. Der Tragarm 14 ist dabei an einer durch eine Bolzen 16 gebildeten Schwenkachse verschwenkbar mit dem ersten Befestigungselement 12 verbunden. Weiterhin weist die Tragarmgelenkvorrichtung 10 ein zweites Befestigungselement 18 auf, mittels welchem die Tragarmgelenkvorrichtung 10 beispielsweise auf einem Tisch befestigt werden kann.

Das zweite Befestigungselement 18 umfasst dabei eine Verstellvorrichtung 20, welche einen Motor 22 und eine Gewindespindel 24 aufweist. Diese Verstellvorrichtung 20 ist dabei mit dem Tragarm 14 nach Art einer Wippe verbunden. Dadurch kann der Tragarm 14 nach oben und unten verschwenkt werden. Eine nach unten verschwenkte Stellung ist dabei beispielsweise in Fig. 2 gezeigt, während Fig. 1 eine waagerechte Stellung zeigt. Die Schwenkachse der Wippe wird dabei durch einen weiteren Lagerbolzen 26 gebildet.

Wie sofort aus Fig. 1 und Fig. 2 ersichtlich ist, ist ein Hebelarm von der Verstellvorrichtung 20 zu dem Lagerbolzen 26 wesentlich kürzer als ein Hebelarm von dem ersten Befestigungselement 12 zu dem Lagerbolzen 26. Entsprechend wird die beispielsweise an dem ersten Befestigungselement 12 gehaltene OP-Lampe durch ihr Eigengewicht ein wesentlich größeres Drehmoment bezüglich des Lagerbolzens 26 erzeugen, als eine vergleichbare durch die Verstellvorrichtung 20 erzeugte Kraft. Entsprechend muss die Verstellvorrichtung 20 bzw. der Motor 22 dazu ausgebildet sein, eine im Vergleich zur zu haltenden Gewichtskraft wesentlich höhere Kraft erzeugen zu können.

Die Bauweise der Tragarmgelenkvorrichtung 10 gemäß Fig. 1 und Fig. 2 erfordert also einen vergleichsweise starken und großen Motor 22. Eine solche Bauweise kann beispielsweise sinnvoll sein, wenn genügend Platz vorhanden ist, der Motor 22 sehr robust sein soll und zudem für eine Wartung leicht erreichbar sein soll. Bei dem gezeigten Beispiel handelt es sich um eine verstellbare Tragarmgelenkvorrichtung 10, deren Verstellung durch eine direkte Kraftansteuerung erfolgt.

Gerade bei beengten Platzverhältnissen, leichteren medizinischen Geräten und filigranen Operationen ist jedoch eine einfache präzise Einstellbarkeit einer Tragarmgelenkvorrichtung bei gleichzeitig geringem Platzbedarf und einer einfachen Bauweise gegebenenfalls vorteilhaft.

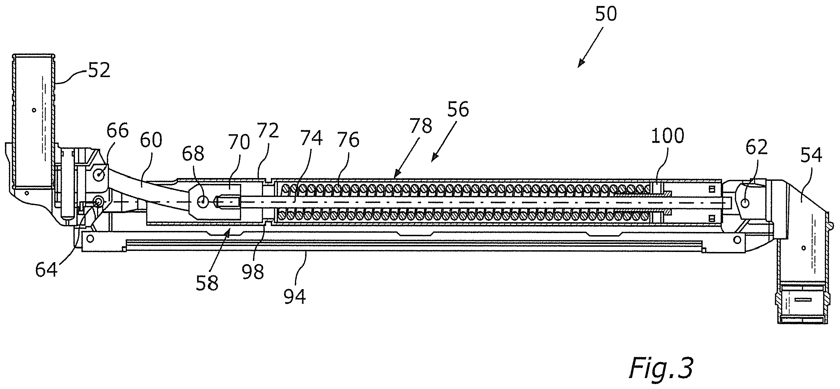

Deshalb wird die Tragarmgelenkvorrichtung 50, wie diese in einer geschnittenen schematischen Seitenansicht in Fig. 3 gezeigt ist, bereitgestellt, welche einen gelenkigen Tragarm bildet. Die Tragarmgelenkvorrichtung 50 weist ein erstes Befestigungselement 52 auf, mittels welchem die Tragarmgelenkvorrichtung 50 beispielsweise an einer Decke eines OP-Saals befestigt werden kann. Alternativ oder zusätzlich kann das erste Befestigungselement 52 beispielsweise auch an einem weiteren Tragarm angeordnet sein. Weiterhin weist die Tragarmgelenkvorrichtung 50 ein zweites Befestigungselement 54 auf, welches beispielsweise zum Befestigen eines medizinischen Geräts, wie eine OP-Lampe, oder auch eines weiteren Tragarms ausgebildet ist. Die beiden Befestigungselement 52, 54 sind dabei mittels eines Federarms 56 miteinander verbunden, wobei der Federarm 56 ein relatives Verschwenken der beiden Befestigungselementen 52, 54 zueinander zulässt. Vorliegend ist die Tragarmgelenkvorrichtung 50 dabei so ausgebildet, dass dieses Verschwenken in einer vertikalen Ebene erfolgt, welche vorliegend der Zeichenebene der Fig. 3 entspricht. Dadurch ermöglicht die Tragarmgelenkvorrichtung 50 bevorzugt eine Höhenverstellung eines daran gehaltenen medizinischen Geräts.

Der Federarm 56 weist dabei einen Stützarm 58 und einen Hebelarm 60 auf. Der Stützarm 58 ist dabei aus mehreren Teilen gebildet, zu welchen unter anderem ein Rohr 72 gehört.

Der Stützarm 58 ist dabei an einem Ende mittels eines Bolzens 64 um eine Stützarmschwenkachse verschwenkbar an dem ersten Befestigungselement 52 gelagert. An einem dazu abgewandten Ende ist der Stützarm 58 ebenfalls mittels eines weiteren Bolzens 62 an dem zweiten Befestigungselement 54 verschwenkbar gelagert. Die Erstreckung von dem Bolzen 64 zu dem Bolzen 62 entspricht dabei der axialen Erstreckung des Stützarms 58.

Der Hebelarm 60 ist vorliegend als Stange ausgebildet und mittels eines weiteren Bolzens 66 an einem Ende verschwenkbar um seine Hebelarmschwenkachse an dem ersten Befestigungselement 52 gelagert. An seinem entgegengesetzten Ende ist der Hebelarm 60 ebenfalls mittels eines Bolzens 68 an einem Schlitten 70 axial beweglich in dem Rohr 72 des Stützarms 58 gelagert. Der Schlitten ist dabei von einer Gewindestange 74 gehalten, um welche radial außen eine Spiralfeder 76 innerhalb des Rohrs 72 angeordnet ist. Der Federarm 56 weist so eine Federvorrichtung 78 auf, mittels welchem jeweilige auf die Tragarmgelenkvorrichtung 50 wirkende Gewichtskräfte abgestützt werden können.

Dadurch dass der Hebelarm 60 und der Stützarm 58 mit ihrer jeweiligen Stützarmschwenkachse bzw. Hebelarmschwenkachse voneinander beabstandet an dem ersten Befestigungselement 52 gelagert sind, ergibt sich ein je nach Stellung der Tragarmgelenkvorrichtung 50 unterschiedlicher Hebelarm bzw. Winkel, mittels welchem der Hebelarm 60 den Stützarm 58 abstützt. So kann bei unterschiedlichen Stellungen der Tragarmgelenkvorrichtung 50 die gleiche Gewichtskraft gleichermaßen abgestützt werden. Es handelt sich also um einen balancierten Federarm 56.

Vorliegend sind die jeweiligen Bolzen 62, 64, 66 and 68 parallel zueinander ausgerichtet. Die jeweiligen Bolzen bilden dabei eine zugeordnete Schwenkachse, welche sich vorliegend orthogonal zu der Papierebene von Fig. 3 erstrecken. Die jeweiligen Bolzen bzw. die dortige jeweiligen Verbindungen von Teilen können deshalb auch als Achse bzw. Lagerung bezeichnet werden.

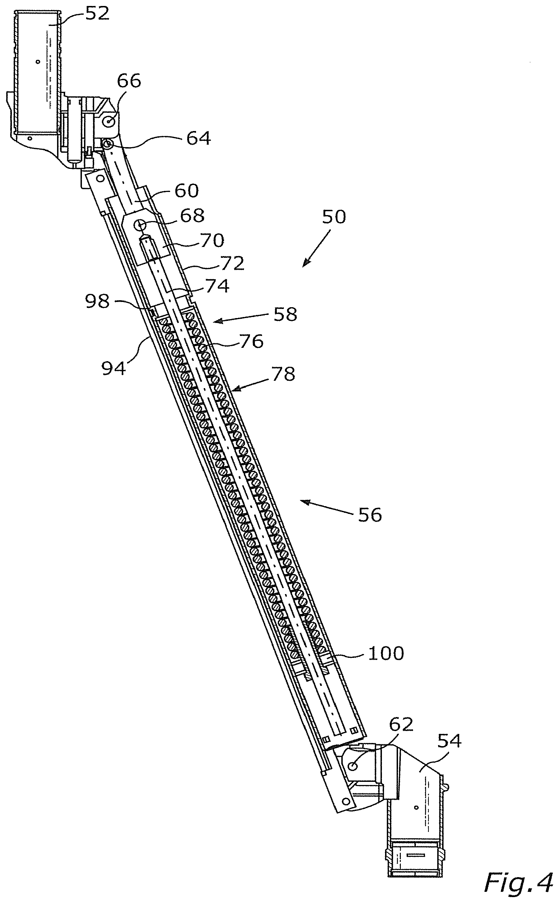

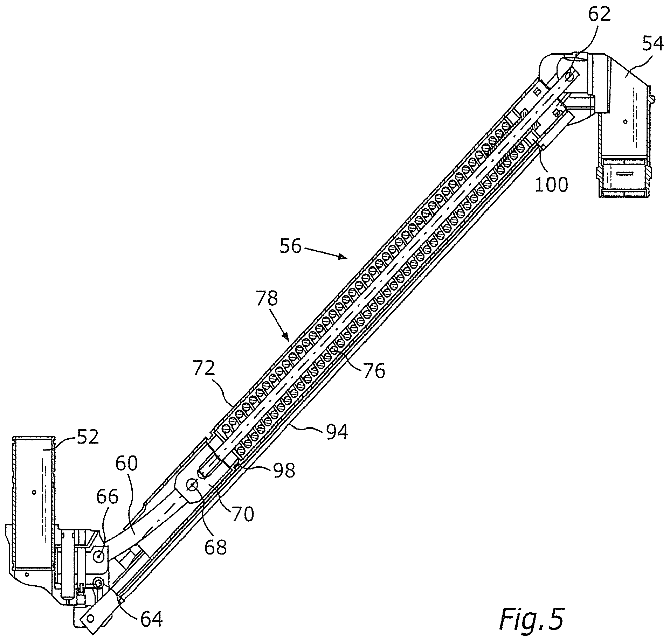

Fig. 4 veranschaulicht eine unterste Stellung der Tragarmgelenkvorrichtung 50, bei welcher der Federarm 56 bzw. das zweite Befestigungselement 54 so weit wie möglich nach unten verschwenkt wurde. Fig. 5 veranschaulicht dagegen eine entgegengesetzte, oberste Extrempositionen des Federarms 56. Jeweilige Richtungen der wirkenden Kräfte und deren jeweiligen zugeordneten Hebelarme zum Bestimmen eines dadurch wirkenden Drehmoments lassen sich so leicht nachvollziehen. Fig. 3 zeigt eine im Wesentlichen waagerechte Stellung der Tragarmgelenkvorrichtung 50. Vorliegend ist der Stützarm 58 beispielsweise 70° gegenüber einer horizontalen Lage in Fig. 4 nach unten verschwenkt und 45° in Fig. 5 nach oben.

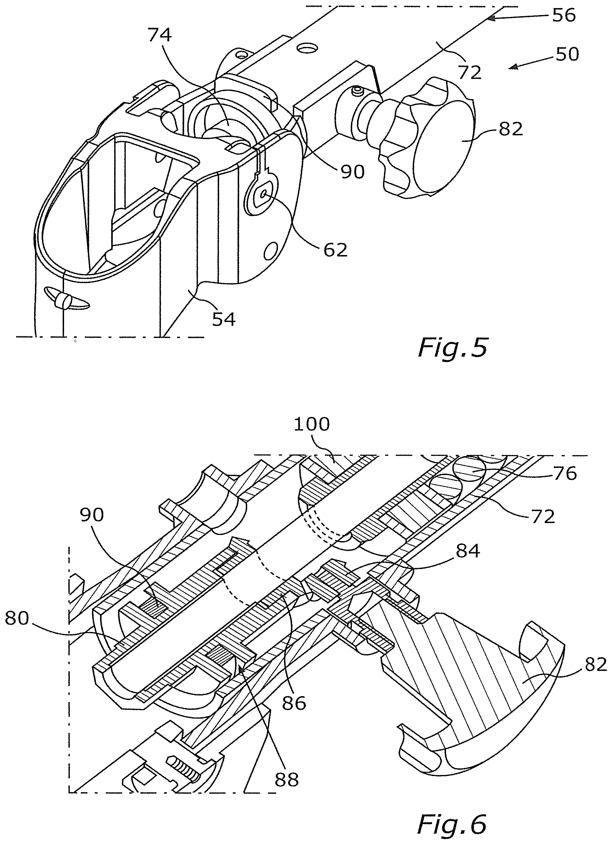

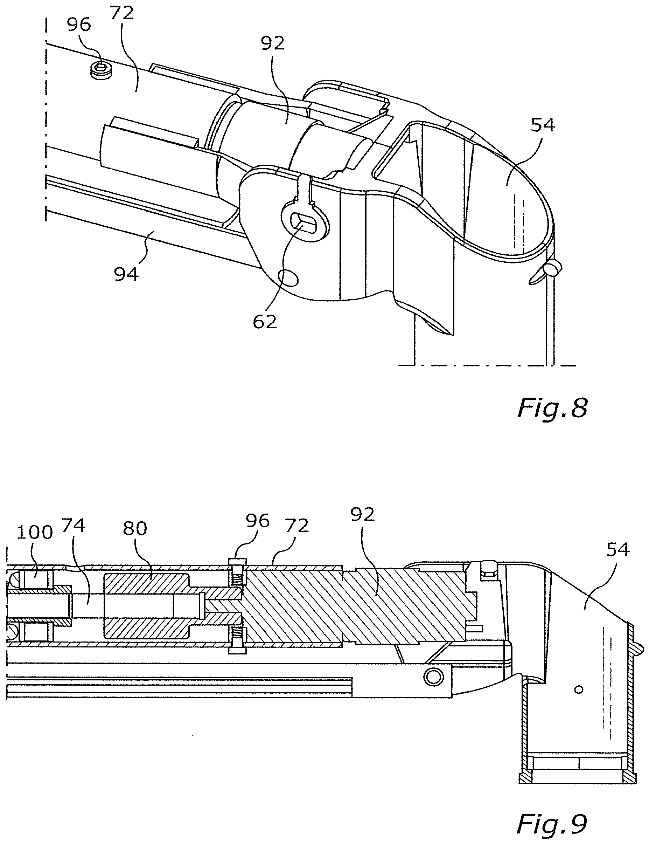

Bei der Tragarmgelenkvorrichtung 50 lässt sich dabei dessen Stellung bzw. der Schwenkwinkel durch ein Verstellen der Gewindestange 74 und damit ein axiales Verschieben des Schlittens 70 bzw. einer Lagerung des Hebelarms 60 an dem Stützarm 58 axial entlang der Längserstreckung des Stützarms 58 einstellen. Zu diesem Zweck ist die Gewindestange 74 mittels einer Mutter 80 an ihrem dem Schlitten 70 abgewandten Ende in dem Stützarm 58 bzw. dem Rohr 72 gelagert. Die Mutter 80 ist in Fig. 7 und 9 gezeigt, in Fig. 3 bis 5 dagegen nicht dargestellt. Diese Mutter 80 ist dabei axial an dem Stützarm 58 festgelegt, kann jedoch um Ihre Längsachse in Umfangsrichtung rotiert werden. Durch ein Rotieren der Mutter 80 wird dabei aufgrund jeweiligen Ineinandergreifens der Gewinde der Mutter 80 und der Gewindestange 74 ein Verschieben der Gewindestange 74 innerhalb des Stützarms 58 bewirkt. Beispielsweise durch ein Verschieben nach rechts, wie in Fig. 5 veranschaulicht, wird so der Schlitten 70 durch die gekoppelte Gewindestange 74 in die korrespondierende Richtung gezogen und entsprechend der Federarm 56 nach oben verstellt. Fig. 4 zeigt dagegen ein Verschieben in die entgegengesetzte Richtung, wodurch der Federarm 56 nach unten gesenkt wird.

Wie dabei in Fig. 4 zu erkennen ist, wird so eine Feder 76 der Federvorrichtung 78 in der nach unten verschwenkten Stellung komprimiert. Dadurch kann eine frei werdende potenzielle Energie beim Absenken eines Geräts mit der Tragarmgelenkvorrichtung 50 in der Federvorrichtung 78 gespeichert werden. Beim Schwenken der Tragarmgelenkvorrichtung entgegen die Schwerkraft nach oben, wie in Fig. 5 gezeigt, wird die Feder 76 entspannt und gibt so die gespeicherte Energie wieder ab. Dadurch wird diese Schwenkbewegung bzw. das Verstellen des Schlittens 70 gestützt, womit jeweilige Stellkräfte beim Aufwärtsschwenken besonders gering sein können. Ebenso kann so auch beim Absenken eine vergleichsweise zum Hochschwenken ähnlich große Kraft benötigt werden, wodurch das Verstellen intuitiv und präziser in beide Richtungen ist. Vorliegend wirkt die Schwerkraft in den Fig. 3 bis 5 in der Bildebene nach unten.

Vorzugsweise ist die Feder 76 vorgespannt verbaut. Insbesondere kann die Feder 76 auch in der obersten, in Fig. 5 gezeigten Stellung noch eine Vorspannung aufweisen, damit eine Abstützung über den gesamten Verstellbereich der Tragarmgelenkvorrichtung 50 ermöglicht wird. Vorliegend ist die Feder 76 als Druckfeder ausgebildet. Alternativ könnte die Feder 76 auch in axialer Richtung des Stützarms 58 an der zum ersten Befestigungselement 52 weisenden Seite des Schlittens 70 statt auf der in axialer Richtung zu dem zweiten Befestigungselement 54 weisenden Seite angeordnet sein. Die Feder 76 ist vorliegend einseitig an einem Anschlag 100 abgestützt, mittels welchem eine Federkraft axial an die Gewindestange 74 übertragbar ist. An ihrem entgegengesetzten Ende ist die Feder 76 axial an einer Verjüngung 98 des Rohrs 72 abgestützt. Zur besseren Montierbarkeit ist dabei eine Einlegscheibe an der Verjüngung 98 angeordnet. Die Verjüngung 98 begrenzt dabei auch eine maximale axiale Bewegung des Schlittens 70. Alternativ könnte sich die Feder 76 auch direkt an dem Schlitten 70 abstützen.

Die Verstellvorrichtung mit der Gewindestange 74 erlaubt durch die jeweiligen Gewinde ein besonders präzises Einstellen der Position der Tragarmgelenkvorrichtung 50. Darüber hinaus kann die Gewindestange 74 ein Übersetzungsverhältnis einer Bedienkraft bereitstellen, wodurch die Tragarmgelenkvorrichtung 50 mit geringem Kraftaufwand bewegt bzw. verstellt werden kann. Zudem ist die Gewindestange 74 selbsthemmend, d. h. bei einem Verlust einer Antriebskraft senkt sich die Tragarmgelenkvorrichtung 50 nicht selbstständig ab. Zudem wird die Verstellvorrichtung durch die Spiralfeder 76 entlastet, wodurch nochmals geringere Bedienkräfte erforderlich werden. Geringe Bedienkräfte erleichtern dabei die Verstellung und ermöglichen eine präzisere Verstellung.

Bei der Tragarmgelenkvorrichtung 50 sind fast alle beweglichen Komponenten der Stützmechanik nahezu vollständig in dem Rohr 72 des Stützarms 58 aufgenommen, wodurch diese zum einen vor Umwelteinflüssen geschützt sind und zudem auch jeweilige Benutzer vor Quetschung durch bewegliche Teile geschützt werden. Außerdem wird so eine sehr kompakte und einfache Bauweise ermöglicht, welche insbesondere auf aufwendige Verkleidungen und Gehäuse verzichten kann.

Fig. 6 veranschaulicht dabei eine manuell verstellbare Ausführungsform der Tragarmgelenkvorrichtung 50 gemäß Fig. 3. Wie zuerkennen ist, weist die Tragarmgelenkvorrichtung 50 gemäß Fig. 6 einen seitlich angeordneten Drehknopf 82 als Stellelement auf. Dieser Drehknopf 82 ist mit zwei Kegelräder 84, 86 mit der Mutter 80 zu deren Rotation verbunden, welche vorliegend beispielsweise als Spindelmutter ausgebildet ist. Diese Mutter 80 weist eine Nut 88 auf, in welcher ein Anschlagelement 90 angeordnet ist. Mittels dieses Anschlagselement 90 ist die Mutter 80 axial in dem Rohr 72 fixiert aber in ihrer Umfangsrichtung drehbar. Zur Fixierung ist dabei das Anschlagelement 90 durch eine Durchgangsöffnung in dem Rohr 72 geführt, wie dies in Fig. 6 zuerkennen ist. Dadurch ist die Tragarmgelenkvorrichtung 50 leicht zu demontieren. Mittels der jeweiligen Kegelräder 84, 86 kann zudem eine weitere Übersetzungsstufe bereitgestellt werden, welche entweder die benötigte Bedienkraft senken kann oder eine Verstellgeschwindigkeit erhöhen kann. Zudem erlauben die jeweiligen Kegelräder 84, 86 den Drehknopf 82 quer zur Längsachse der Gewindestange 74 anzuordnen, wodurch die Tragarmgelenkvorrichtung 50 besonders kompakt sein kann.

Fig. 8 veranschaulicht dagegen eine Ausführungsform der Tragarmgelenkvorrichtung 50 gemäß Fig. 3, bei welcher eine Verstellung mittels eines Motors 92 erfolgt. Dieser Motor 92 ist vorliegend als elektrischer Getriebemotor ausgeführt, welcher in einer axialen Verlängerung des Stützarms 58 angeordnet ist. Der Motor 92 ist, wie in der Schnittansicht gemäß Fig. 9 zuerkennen ist, mittels jeweiliger Klemmschrauben 94 an dem Rohr 72 befestigt und mit der Mutter 80 verklemmt und so zur Kraftübertragung mit der Gewindestange 74 verbunden. Die Mutter 80 ist auch bei dieser Ausführungsform axial fixiert, aber in Umfangsrichtung drehbar. Die Mutter 80 kann dabei analog zu der Ausführungsform gemäß Fig. 6 fixiert sein oder aber auch beispielsweise an dem Motor 92. Alternativ könnte der Motor 92 aber auch direkt mit der Gewindestange 74 zu deren Verstellung bzw. Drehen verbunden sein. Wie in Fig. 9 zuerkennen ist, kann auch der Motor 92 wenigstens teilweise in dem Rohr 72 angeordnet sein, da dieser aufgrund der Bauweise der Tragarmgelenkvorrichtung 50 nur ein geringes Drehmoment und eine geringe Leistung zur Verfügung stellen muss. Dadurch kann die Tragarmgelenkvorrichtung 50 besonders kompakt und einfach sein.

Zudem ist in den Fig. 3 bis 5, Fig. 8 und Fig. 9 zuerkennen, dass die Tragarmgelenkvorrichtung 50 ein Parallelführungselement 94 aufweisen kann. Das Parallelführungselement 94 ist dabei vorliegend als Stange ausgebildet, welche verschwenkbar an dem ersten Befestigungselement 52 mit einem ersten Enden und verschwenkbar mit einem zweiten Ende an dem zweiten Befestigungselement 54 gelagert ist. Durch das Parallelführungselement 94 wird eine Nickbewegung der beiden Befestigungselemente 52, 54 mit einer Verschwenkbewegung des Federarms 56 synchronisiert. Dadurch kann eine Ausrichtung der beiden Befestigungselemente 52, 54 bei allen Stellung des Federarms 56 gleich bleiben. Dies ist unmittelbar durch einen Vergleich der Fig. 3, Fig. 4 und Fig. 5 miteinander zu erkennen, bei welchem die Ausrichtung der beiden Befestigungselemente 52, 54 in der Bild- bzw. Zeichenebene gleich bleibt.

### Bezugszeichenliste

- 10: Tragarmgelenkvorrichtung
- 12: erstes Befestigungselement
- 14: Tragarm
- 16: Bolzen
- 18: zweites Befestigungselement
- 20: Verstellvorrichtung
- 22: Motor
- 24: Gewindespindel
- 26: Lagerbolzen

- 50: Tragarmgelenkvorrichtung
- 52: erstes Befestigungselement
- 54: zweites Befestigungselement
- 56: Federarm
- 58: Stützarm
- 60: Hebelarm
- 62: Bolzen
- 64: Bolzen (Stützarmschwenkachse)
- 66: Bolzen (Hebelarmschwenkachse)
- 68: Bolzen
- 70: Schlitten
- 72: Rohr
- 74: Gewindestange
- 76: (Spiral)feder
- 78: Federvorrichtung
- 80: Mutter
- 82: Drehknopf (Stellelement)
- 84: Kegelrad
- 86: Kegelrad
- 88: Nut
- 90: Anschlagelement
- 92: Motor
- 94: Parallelführungselement
- 96: Klemmschraube
- 98: Verjüngung
- 100: Anschlag

## Patentansprüche

1. Tragarmgelenkvorrichtung (50) für ein Tragsystem zum bewegbaren Halten wenigstens eines medizinischen Geräts,
umfassend ein erstes und ein zweites Befestigungselement (52, 54), welche mittels eines Federarms (56) relativ verschwenkbar zueinander miteinander verbunden sind, und
wobei der Federarm (56) einen Stützarm (58) und einen Hebelarm (60) aufweist, welche mit einer zugeordneten Stützarmschwenkachse (64) und Hebelarmschwenkachse (66) an dem ersten Befestigungselement (52) verschwenkbar gelagert sind,
wobei die beiden Schwenkachsen (64, 66) voneinander beabstandet sind,
wobei der Stützarm (58) an dem zweiten Befestigungselement (54) verschwenkbar gelagert ist und der Hebelarm (60) mittels einer Federvorrichtung (78) an dem Stützarm (58) derart gelagert ist, dass eine Gewichtskraft des gehaltenen medizinischen Geräts bei unterschiedlichen Schwenkpositionen abgestützt wird,
wobei die Federvorrichtung (78) eine Verstellvorrichtung aufweist, mittels welcher eine Position der Lagerung (68, 70) des Hebelarms (60) an dem Stützarm (58) entlang einer Längserstreckung des Stützarms (58) einstellbar ist.

2. Tragarmgelenkvorrichtung (50) nach Anspruch 1,
wobei die Federvorrichtung (78) eine Feder (76) aufweist, welche vorzugsweise im Wesentlichen innerhalb des Stützarms (58) geführt ist.

3. Tragarmgelenkvorrichtung (50) nach Anspruch 1 oder 2,
wobei der Hebelarm (60) verschwenkbar an der Federvorrichtung (78) gelagert ist, insbesondere mit einer Schwenkachse (68) parallel zu der Hebelarmschwenkachse (66), mittels welcher der Hebelarm (60) an dem ersten Befestigungselement (52) gelagert ist.

4. Tragarmgelenkvorrichtung (50) nach einem der vorhergehenden Ansprüche,
wobei der Hebelarm (60) mittels eines Schlittens (70) an dem Stützarm (58) entlang dessen Längserstreckung gelagert ist, wobei der Schlitten (70) vorzugsweise im Wesentlichen innerhalb des Stützarms (58) geführt ist.

5. Tragarmgelenkvorrichtung (50) nach einem der vorhergehenden Ansprüche,
wobei die Verstellvorrichtung eine Gewindestange (74) aufweist, durch deren Verschieben die Position der Lagerung (68, 70) des Hebelarms (60) am Stützarm (58) einstellbar ist.

6. Tragarmgelenkvorrichtung (50) nach Anspruch 5,
wobei die Gewindestange (74) an einer axial befestigten und drehbaren Mutter (80) gelagert ist, welche in oder an dem Stützarm (58) befestigt ist

7. Tragarmgelenkvorrichtung (50) nach einem der vorhergehenden Ansprüche,
wobei die Verstellvorrichtung ein Stellelement (82) aufweist, mittels welchem die Position der Lagerung (68, 70) des Hebelarms (60) an dem Stützarm (58) einstellbar ist, wobei das Stellelement (82) bevorzugt an dem Stützarm (58) gelagert ist.

8. Tragarmgelenkvorrichtung (50) nach einem der vorhergehenden Ansprüche,
wobei die Verstellvorrichtung einen Motor (92) aufweist, mittels welchem die Position der Lagerung (68, 70) des Hebelarms (60) an dem Stützarm (58) einstellbar ist, wobei der Motor (92) bevorzugt an dem Stützarm (58) gelagert ist.

9. Tragarmgelenkvorrichtung (50) nach Anspruch 8,
wobei die Tragarmgelenkvorrichtung (50) eine Sensorvorrichtung aufweist, welche dazu ausgebildet ist, eine auf die Tragarmgelenkvorrichtung (50) wirkende Kraft und/oder eine Stellung der beiden Befestigungselemente (52, 54) relativ zueinander zu erfassen, und
wobei die Tragarmgelenkvorrichtung (50) eine Steuervorrichtung aufweist, welche in Abhängigkeit von der erfassten auf die Tragarmgelenkvorrichtung (50) wirkende Kraft und/oder der erfassten Stellung der Tragarmgelenkvorrichtung (50) den Motor (92) zum Halten der relativen Stellung der beiden Befestigungselemente (52, 54) zueinander steuert.

10. Tragarmgelenkvorrichtung (50) nach einem der vorhergehenden Ansprüche,
wobei die Tragarmgelenkvorrichtung (50) ein Parallelführungselement (94) aufweist, welches jeweils schwenkbar an dem ersten Befestigungselement (52) und dem zweiten Befestigungselement (54) beabstandet zu dem Stützarm (58) gelagert ist.

11. Tragarmgelenkvorrichtung (50) nach einem der vorhergehenden Ansprüche,
wobei jeweilige Lagerachsen des Parallelführungselements (94) parallel zueinander und/oder zu jeweiligen Lagerachsen des Stützarms (58) sind.

12. Tragarmgelenkvorrichtung (50) nach einem der vorhergehenden Ansprüche,
wobei die Tragarmgelenkvorrichtung (50) dazu ausgebildet ist, eine Stützkraft an unterschiedliche Gewichtskräfte unterschiedlicher gehaltener medizinischer Geräte bei unterschiedlichen Schwenkpositionen anzupassen, insbesondere durch Austausch der Feder (76) der Federvorrichtung (78), Einstellen einer Vorspannkraft der Federvorrichtung (78) und/oder Einstellen eines Abstands zwischen der Hebelarmschwenkachse (66) und der Stützarmschwenkachse (64) an dem ersten Befestigungselement (52).

13. Tragarmgelenkvorrichtung (50) nach einem der vorhergehenden Ansprüche,
wobei das erste Befestigungselement (52) und/oder das zweite Befestigungselement (54) zur Befestigung an einer Wand, einer Decke, einem Boden, einer beweglichen und/oder einer stationären Basiseinheit ausgebildet ist/sind
und/oder
wobei das erste Befestigungselement (52) und/oder das zweite Befestigungselement (54) zur Kopplung mit einem Tragarm, einem Gelenk, einer weiteren Tragarmgelenkvorrichtung (50) und/oder dem bzw. einem medizinischen Gerät ausgebildet ist/sind.

14. Tragarmsystem zum bewegbaren Halten wenigstens eines medizinischen Geräts umfassend wenigstens eine Tragarmgelenkvorrichtung (50) nach einem der vorhergehenden Ansprüche.

15. Verfahren zum Betreiben einer Tragarmgelenkvorrichtung (50) und/oder eines Tragarmsystems gemäß einer der vorhergehenden Ansprüche in deren Rückbezug auf die Tragarmgelenkvorrichtung (50) gemäß Anspruch 8 oder 9, wenigstens die folgenden Schritte umfassend:
• Bereitstellen der Tragarmgelenkvorrichtung (50) und/oder des Tragarmsystems, insbesondere mit daran befestigtem medizinischem Gerät;
• Erfassen einer auf die Tragarmgelenkvorrichtung (50) wirkende Kraft und/oder einer Stellung der beiden Befestigungselemente (52, 54) relativ zueinander; und
• Steuern des Motors (92) der Tragarmgelenkvorrichtung (50) in Abhängigkeit von der erfassten auf die Tragarmgelenkvorrichtung (50) wirkende Kraft und/oder der erfassten Stellung der Tragarmgelenkvorrichtung (50) zum Halten der relativen Stellung der beiden Befestigungselemente (52, 54) zueinander.
